(19) Europäisches Patentamt
European Patent Office
Office européen des brevets

(11) Veröffentlichungsnummer : **0 055 359**
**B1**

(12) **EUROPÄISCHE PATENTSCHRIFT**

(45) Veröffentlichungstag der Patentschrift : **24.08.83**

(51) Int. Cl.³ : **C 07 C119/042**, C 07 C118/00// C07C125/065

(21) Anmeldenummer : **81108880.6**

(22) Anmeldetag : **24.10.81**

# (54) Verfahren zur Herstellung von 1-Alkenylisocyanaten.

(30) Priorität : **31.12.80 DE 3049627**

(43) Veröffentlichungstag der Anmeldung : **07.07.82 Patentblatt 82/27**

(45) Bekanntmachung des Hinweises auf die Patenterteilung : **24.08.83 Patentblatt 83/34**

(84) Benannte Vertragsstaaten : **BE CH DE FR GB IT LI**

(56) Entgegenhaltungen :
**DE A 2 512 514**
**DE A 2 756 928**
**DE B 1 922 412**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Hutmacher, Hans-Martin, Dr.**
**Neckarpromenade 16**
**D-6800 Mannheim (DE)**
Erfinder : **Towae, Friedrich, Dr.**
**Parkstrasse 22**
**D-6700 Ludwigshafen (DE)**

**EP 0 055 359 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schrifflich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Verfahren zur Herstellung von 1-Alkenylisocyanaten

Die Erfindung betrifft ein Verfahren zur Herstellung von 1-Alkenylisocyanaten durch Umsetzung von Aldehyden mit Carbamaten zu N-(1-Alkenyl)-carbamaten und deren Spaltung bei erhöhter Temperatur.

Die Herstellung von Isocyanaten durch thermische Zersetzung von Harnstoffen und Carbamaten ist bekannt (Houben-Weyl, Methoden der Organischen Chemie, Band 8, Seiten 126 bis 128 (1952)). Die Spaltung N-ungesättigt substituierter Carbamate zu N-Alkenylisocyanaten ist jedoch nicht beschrieben. In DE-OS 2 512 514 wird die Zersetzung von Naphthylcarbamaten und in DE-OS 2 756 928 die Flüssigphasenspaltung von Arylcarbamaten empfohlen. Beide Verfahren gehen von Ausgangsstoffen aus, die wirtschaftlich nur über Phosgenierungsreaktionen hergestellt werden können. Beispiele mit Spaltung zu 1-Alkenylisocyanaten werden nicht angegeben.

Bei der Herstellung von 1-Alkenylisocyanaten durch katalytische Zersetzung von N-tert.-Alkyl-N-(1-alkenyl)-carbamidsäurechloriden (DE-AS 1 922 412) sind Vorstufen notwendig, zu deren Herstellung mit Phosgen gearbeitet werden muß. Ferner ist es bekannt, 1-Alkenylisocyanate durch Curtiusabbau von Aziden herzustellen (J. Org. Chem., *26,* 770-779 (1961)). Neben den hohen Einsatzstoffkosten sprechen insbesondere sicherheitstechnische Gründe gegen diesen Syntheseweg.

Es wurde nun gefunden, daß man 1-Alkenylisocyanate der Formel

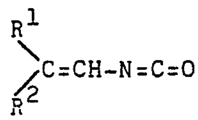
(I)

worin $R^1$ und $R^2$ gleich oder verschieden sein können und jeweils ein Wasserstoffatom, ein Halogenatom, einen aliphatischen, cycloaliphatischen, araliphatischen oder aromatischen Rest bedeuten, $R^1$ und $R^2$ zusammen mit dem benachbarten Kohlenstoffatom auch Glieder eines Ringes bezeichnen, vorteilhaft herstellt, wenn man einen Aldehyd der Formel

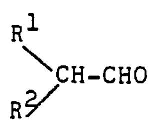
(II)

worin $R^1$ und $R^2$ die vorgenannte Bedeutung besitzen, mit einem Carbamat der Formel

$$H_2N—CO_2R^3 \qquad (III)$$

worin $R^3$ einen aliphatischen, cycloaliphatischen oder araliphatischen Rest bedeutet, zu einem N-(1-Alkenyl)-carbamat der Formel

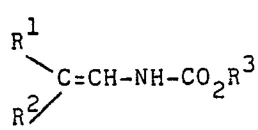
(IV)

worin $R^1$, $R^2$ und $R^3$ die vorgenannten Bedeutungen besitzen, umsetzt und den Stoff IV bei Temperaturen von 250 bis 600 °C erhitzt.

Die Umsetzung kann für den Fall der Verwendung von 2-Phenylpropanal und Methylcarbamat durch die folgenden Formeln wiedergegeben werden :

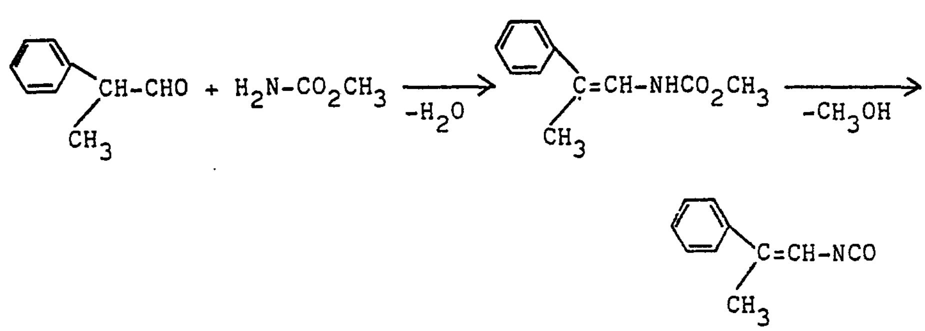


Im Hinblick auf den Stand der Technik liefert das Verfahren nach der Erfindung überraschend auf

einfacherem und wirtschaftlicherem Wege 1-Alkenylisocyanate in besserer Ausbeute und Reinheit, aus wirtschaftlich gut zugänglichen Ausgangsstoffen auf sicherheitstechnisch unbedenkliche Weise, d. h. insbesondere unter Vermeidung von Phosgen oder Aziden bei der Bereitung von Zwischenstufen. Diese vorteilhaften Eigenschaften des erfindungsgemäßen Verfahrens waren nicht vorauszusehen, denn es ist bekannt, daß es sich bei 1-Alkenylisocyanaten um relativ labile Verbindungen handelt, so daß ihre Herstellung durch Carbamatspaltung (Thermolyse) bislang nicht in Betracht kam. Die gut durchführbare Spaltung der beanspruchten O-Alkyl-N-(1-alkenyl)-carbamate ist überraschend gegenüber der Lehre von DE-OS 2 756 928 Seite 5, daß Arylcarbamate im Vergleich zu Alkylcarbamaten unter milderen Bedingungen spalten.

Bevorzugte Ausgangsstoffe II, III, Stoffe IV und demzufolge bevorzugte Endstoffe I sind solche, in deren Formeln die Reste $R^1$, $R^2$ und $R^3$ gleich oder verschieden sein können und einen Alkylrest mit 1 bis 12, insbesondere 1 bis 8 Kohlenstoffatomen, einen Cyclohexylrest, einen Aralkylrest mit 7 bis 12 Kohlenstoffatomen bezeichnen, $R^1$ und $R^2$ auch jeweils für einen Alkylarylrest mit 7 bis 12 Kohlenstoffatomen oder einen Phenylrest, ein Wasserstoffatom, ein Bromatom oder insbesondere ein Chloratom stehen, $R^1$ und $R^2$ zusammen mit dem benachbarten Kohlenstoffatom auch Glieder eines 5- oder 6-gliedrigen alicyclischen Ringes bezeichnen. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Geeignete Ausgangsstoffe II sind beispielsweise : Acetaldehyd, Propionaldehyd, n-Butyraldehyd, iso-Butyraldehyd, Dichloracetaldehyd, Chloracetaldehyd, α-Chlorpropionaldehyd, Cyclohexylacetaldehyd, Phenylpropionaldehyd, Phenyläthylaldehyd, 2-Methylbutyraldehyd, 2-Äthylcapronaldehyd, n-Valeraldehyd, Isovaleraldehyd, n-Capronaldehyd, Isocapronaldehyd, 2-Methyl-valeraldehyd, 3-Methyl-valeraldehyd, 2-Äthylbutyraldehyd, 2,3-Dimethylbutyraldehyd, 3,3-Dimethylbutyraldehyd, Önanthaldehyd, 2-Methylcapronaldehyd, 3-Methyl-capronaldehyd, 4-Methyl-capronaldehyd, 5-Methyl-capronaldehyd, 2-Äthyl-valeraldehyd, 3-Äthyl-valeraldehyd, 3,3-Dimethylvaleraldehyd, 2,3-Dimethylvaleraldehyd, 4-Äthylvaleraldehyd, 4,4-Dimethylvaleraldehyd, 3,4-Dimethylvaleraldehyd, 2,4-Dimethylvaleraldehyd, 2-Äthyl-2-methyl-butyraldehyd, 2-Äthyl-3-methyl-butyraldehyd. Es kommen bevorzugt in Betracht : Acetaldehyd, n-Propanal, n-Butanal, i-Butyraldehyd, n-Pentanal, 2-Methyl-butanal, 2-Methyl-pentanal, 2-Phenyl-propanal, 2-Äthylhexanal, 2,3-Dimethylbutanal und Cyclohexylacetaldehyd.

Geeignete Ausgangsstoffe III sind beispielsweise : Octyl-, Nonyl-, Decyl-, Äthyl-carbamat, Propylcarbamat, Isopropylcarbamat, Butyl-, Undecyl-, Dodecylcarbamat, Isobutylcarbamat, sek.-Butylcarbamat, Pentylcarbamat, 3-Methyl-butylcarbamat, Hexylcarbamat, 2-Äthylhexylcarbamat, Cyclohexylcarbamat, Cyclopentylcarbamat, Benzylcarbamat, Methylcarbamat, Heptylcarbamat.

Die unsubstituierten aliphatischen Carbamate der Formel III sind im Gegensatz zu aromatischen Carbamaten (Chem. Rev. *65,* 570 (1965)) auf einfache Weise ohne Verwendung von Phosgen aus Harnstoff und entsprechenden Alkoholen zugänglich. Bevorzugt sind Methyl-, Ethyl-, n-Butyl-, n-Octylcarbamat, Benzylcarbamat.

Die Umsetzung des 1. Schrittes, der Herstellung des Ausgangsstoffs IV, wird vorteilhaft bei einer Temperatur von − 20 bis + 150 °C, vorzugsweise zwischen 0 und 100 °C, insbesondere von 10 bis 60 °C, drucklos oder unter Druck, diskontinuierlich oder kontinuierlich durchgeführt. Zweckmäßig verwendet man unter den Reaktionsbedingungen inerte Lösungsmittel. Es kann aber auch ohne Lösungsmittel gearbeitet werden. Als Lösungsmittel kommen z. B. in Frage : aromatische Kohlenwasserstoffe, z. B. Toluol, Äthylbenzol, o-, m-, p-Xylol, Isopropylbenzol ; Halogenkohlenwasserstoffe, insbesondere Chlorkohlenwasserstoffe, z. B. Tetrachloräthylen, 1,1,2,2- oder 1,1,1,2-Tetrachloräthan, Amylchlorid, Dichlorpropan, Methylenchlorid, Dichlorbutan, Chloroform, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, Chlorbenzol, o-, p- und m-Dichlorbenzol, o-, m-, p-Chlortoluol, 1,2,4-Trichlorbenzol ; Äther, z. B. Äthylpropyläther, n-Butyläthyläther, Di-n-butyläther, Diisobutyläther, Diisoamyläther, Diisopropyläther, Cyclohexylmethyläther, Diäthyläther, Äthylenglykoldimethyläther, Tetrahydrofuran, Dioxan ; Alkanole und Cycloalkanole wie Äthanol, Methanol, n-Butanol, Isobutanol, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Metnylcyclohexanol, insbesondere solche mit 1 bis 8 Kohlenstoffatomen ; aliphatische oder cycloaliphatische Kohlenwasserstoffe, z. B. Heptan, Nonan, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan ; und entsprechende Gemische. Zweckmäßig verwendet man das Lösungsmittel in einer Menge von 50 bis 10 000 Gewichtsprozent, vorzugsweise von 100 bis 500 Gewichtsprozent, bezogen auf Ausgangsstoff II.

Die Umsetzung wird vorteilhaft in Gegenwart von Säure als Katalysator, vorteilhaft mit einer Menge von 0,000 1 bis 0,1, insbesondere von 0,001 bis 0,05 Äquivalenten Katalysator, bezogen auf 1 Mol Ausgangsstoff II, durchgeführt. Es können anorganische oder organische Säuren verwendet werden. Anstelle einbasischer Säuren können auch äquivalente Mengen mehrbasischer Säuren zur Anwendung gelangen. Beispielsweise sind folgende Säuren geeignet : Chlorwasserstoff, Bromwasserstoff, Perchlorsäure, Schwefelsäure, Phosphorsäure, Salpetersäure ; Sulfonsäuren wie Benzol- und p-Toluolsulfonsäure ; Bor enthaltende Säuren wie Borsäure, Borfluorwasserstoffsäure ; aliphatische Carbonsäuren wie Chloressigsäure, Dichloressigsäure, Trichloressigsäure, Oxalsäure, Ameisensäure, Cyanessigsäure, Essigsäure, Propionsäure, Buttersäure, Isobuttersäure, Glykolsäure, Milchsäure, Brenztraubensäure, Weinsäure, Zitronensäure, α- bzw. β-Chlorpropionsäure, Bernsteinsäure, Isovaleriansäure, Valeriansäure, Glutarsäure, Adipinsäure, Maleinsäure ; Benzoesäure, 2,3-, 2,4-, 2,5-, 2,6-Dimethylbenzoesäure, o-, m- bzw. p-Oxybenzoesäure, Phenylpropionsäure, Cyclohexancarbonsäure ; oder entsprechende Gemische. Die Säuren können in konzentrierter Form, im Gemisch miteinander und/oder mit einem Lösungsmittel, insbesondere Wasser, angewendet werden. Bevorzugt sind Salzsäure, Schwefelsäure, Phosphorsäure, p-Toluolsulfonsäure, Monochloressigsäure, Di-, Trichloressigsäure.

Die Reaktion kann wie folgt durchgeführt werden : Ein Gemisch von Lösungsmittel, Säure und Ausgangsstoffen II und III wird während 0,5 bis 25 Stunden bei der Reaktionstemperatur gehalten und anschließend fraktionierend destilliert. Der Stoff IV kann isoliert oder direkt in den Reaktor des 2. Schrittes zur Herstellung von Isocyanaten eingeführt werden.

Die Reaktion des 2. Schrittes, die Spaltung, erfolgt durch Erhitzen von IV auf eine Temperatur des angegebenen Bereichs. Dann wird der Endstoff I aus dem Gemisch in üblicher Weise, z. B. durch fraktionierte Destillation, abgetrennt. Die Spaltreaktion wird zweckmäßig so durchgeführt, daß man das N-(1-Alkenyl)-carbamat IV direkt in den Reaktor einführt, oder zunächst in einer dem Pyrolysereaktor vorgeschalteten Verdampferzone verdampft und die Dämpfe anschließend durch den Reaktor leitet. Die aus dem Reaktor austretenden Dämpfe werden einer Trennvorrichtung zugeführt, die es erlaubt, die entstandenen Endstoffe I von dem Alkohol $R^3OH$ abzutrennen, um eine Rekombination zu vermeiden. Als Trennvorrichtung eignet sich z. B. eine Einrichtung zur fraktionierenden Kondensation. Eine gute Ausführungsform ist auch z. B. eine fraktionierte Destillation über eine Kolonne, wobei ein inertes Lösungsmittel als Zwischensieder verwendet werden kann. Die Endstoffe können, falls erforderlich, anschließend z. B. durch Destillation oder Kristallisation gereinigt bzw. von eventuell vorhandenen nicht umgesetztem Ausgangsstoff abgetrennt werden. Als Pyrolysereaktoren sind Vorrichtungen geeignet, mit denen die Carbamate bzw. deren Dämpfe möglichst rasch auf die gewünschte Temperatur gebracht werden können, z. B. beheizte Röhren, die mit als Wärmeträgern dienenden Füllkörpern beschickt sind oder Wirbelschichten.

Die Spaltung wird bei einer Temperatur von 250 bis 600, vorzugsweise von 350 bis 550 °C, insbesondere von 400 bis 550 °C, insbesondere von 400 bis 500 °C, drucklos, mit Druck oder unter Unterdruck, vorzugsweise bei 0,1 bis 1 033 mbar, insbesondere 0,5 bis 500 mbar, vorteilhaft 1 bis 200 mbar, zweckmäßig kontinuierlich oder auch diskontinuierlich durchgeführt. Sie kann unter Zusatz von Inertgasen, z. B. Stickstoff, oder in Gegenwart inerter Lösungsmittel, insbesondere solcher Lösungsmittel, deren Siedepunkt zwischen dem des Endstoffs I und der Alkoholkomponente $R^3OH$ liegt (Zwischensieder), durchgeführt. Die Durchsätze liegen zweckmäßig bei 0,05 bis 0,5 kg Carbamat IV pro Liter und Stunde und sind abhängig von der gewählten Spalttemperatur sowie dem angestrebten Umsatz. Nichtumgesetztes Ausgangsmaterial wird zurückgeführt.

Die Spaltreaktion kann auch unter Druck, Normaldruck oder vermindertem Druck in der Flüssigphase durchgeführt werden, wobei man z. B. das Carbamat IV unter Rühren in einem Reaktor gibt, in dem sich als Wärmeträger ein hochsiedendes, inertes Lösungsmittel wie z. B. Öle vom Kp 300 bis 400 °C, befindet. Trennung und Aufarbeitung der aus dem Reaktor austretenden Dämpfe erfolgt wie bei der zuvor beschriebenen Spaltung. Bevorzugter Temperaturbereich für Flüssigphasenspaltungen ist 250 bis 400 °C.

Als geeignete N-(1-Alkenyl)-carbamate IV kommen beispielsweise alle die in Betracht, die aus den als geeignet genannten Ausgangsstoffen II und III hergestellt wurden, wie Methyl-N-vinylcarbamat, Ethyl-N-vinylcarbamat, Ethyl-N-(1-propenyl)-carbamat, Ethyl-N-(1-butenyl)-carbamat, Ethyl-N-(2-methyl-1-propenyl)-carbamat, Ethyl-N-(2-methyl-1-butenyl)-carbamat. Besonders eignen sich solche N-(1-Alkenyl)-carbamate, bei denen die Siedepunktsunterschiede der entstehenden Endstoffe I und Alkohole eine einfache Trennung erlauben, z. B. : n-Butyl-N-vinylcarbamat, Methyl-N-(1-pentenyl)-carbamat, n-Octyl-N-(2-methyl-1-propenyl)-carbamat, Ethyl-N-(2-methyl-1-pentenyl)-carbamat, Methyl-N-(2-phenyl-1-propenyl)-carbamat.

Die erfindungsgemäß herstellbaren 1,2-ungesättigten Isocyanate I sind wertvolle Ausgangsstoffe für die Herstellung von Schädlingsbekämpfungsmitteln, Farbstoffen, Pharmazeutika, Textilhydrophobierungsmitteln, Waschmitteln, Kunststoffen, Bleichmitteln und Klebstoffen, da sie neben einer reaktiven Isocyanatgruppe noch eine aktivierte Doppelbindung bzw. ein aktiviertes α-C-Atom besitzen. Zudem sind 1-Alkenylisocyanate wichtige Monomere, die in vielfältiger Weise zu Ketten- und Leiterpolymeren, wie z. B. strahlungshärtenden Lackharzen, umgesetzt werden können (Chem. High Polymers (Tokyo) *13* (1956), Seite 390 ; J. Polymer Sc. *35* (1959), Seite 215, J. Org. Chem. *26* (1961), Seite 770 ; J. of Coatings Techn. *49* (1977), Seite 82). Sie können zu Urethanen, z. B. für die Verwendung als Schaumstoffe oder hochmolekulare Überzüge mit hoher Flexibilität, oder Harnstoffen umgesetzt werden. Bezüglich der Verwendung wird auf vorgenannte Veröffentlichungen und Ullmanns Encyklopädie der technischen Chemie, Band 9, Seiten 11, 12, 404 sowie Band 17, Seite 204 (3. Auflage), hingewiesen.

Die in den folgenden Beispielen aufgeführten Teile bedeuten Gewichtsteile. Sie verhalten sich zu den Volumenteilen wie Kilogramm zu Liter.

Beispiel 1

a) Zu einer Lösung von 450 Teilen Methylcarbamat in 1 500 Volumenteilen Diethylether werden bei 25 °C nacheinander unter Rühren 402 Teile 2-Phenylpropanal und 5 Teile p-Toluolsulfonsäure zugegeben. Nach 45 Minuten Reaktionszeit erhält man durch anschließende fraktionierte Destillation des angefallenen Festgutes 468,6 Teile (81,8 % der Theorie, bezogen auf eingesetztes 2-Phenylpropanal) Methyl-N-(2-phenyl-1-propenyl)-carbamat als cis-trans-Isomerengemisch vom Kp 131-136 °C/0,4 mbar sowie 214,3 Teile Methylcarbamat, das wiederverwendet werden kann.

b) Das nach Beispiel 1a) erhaltene Methyl-N-(2-phenyl-1-propenyl)-carbamat wird bei 200 °C/4 mbar kontinuierlich in einem aus einem elektrisch beheizten Quarzrohr bestehenden Verdampfer verdampft und die Dämpfe bei 500 °C/4 mbar durch ein mit 250 Volumenteilen $V_2A$-Maschendrahtringen (∅ 3 mm) gefülltes, elektrisch beheiztes Quarzrohr geführt. Aus den austretenden Dämpfen werden zunächst bei ca 10 °C (Wasserkühlung) 2-Phenyl-1-propenylisocyanat und unumgesetzter Stoff IV auskondensiert, während sich das gebildete Methanol bei − 78 °C in einer nachgeschalteten Trockeneiskühlfalle abscheidet. Nach insgesamt 15 Stunden erhält man durch fraktionierte Destillation des Kondensats 83,9 Teile Stoff IV (82,1 % Umsatz) und 273,5 Teile 2-Phenyl-1-propenylisocyanat (70,1 % Ausbeute, bezogen auf ein- bzw. 85,3 % Ausbeute, bezogen auf umgesetztes Methyl-N-(2-phenyl-1-propenyl)-carbamat) als cis-trans-Isomerengemisch vom Kp 62 bis 67 °C/0,5 mbar.

Beispiel 2

a) Zu einer Lösung von 605,5 Teilen n-Octylcarbamat in 1 750 Volumenteilen Diethylether werden bei 25 °C nacheinander 126 Teile Isobutyraldehyd und 7 Teile p-Toluolsulfonsäure zugegeben, wonach die Temperatur für eine Stunde auf 40 °C ansteigt. Nach einem Tag wird das auskristallisierte Festprodukt abgesaugt und in Gegenwart von 10 Teilen p-Toluolsulfonsäure und einem Teil Hydrochinon fraktioniert destilliert. Dabei erhält man 298,3 Teile n-Octyl-N-(2-methyl-1-propenyl)-carbamat (75,1 % Ausbeute, bezogen auf eingesetzten Isobutyraldehyd) vom Kp 126 °C/1 mbar sowie 279,5 Teile n-Octylcarbamat zurück, die wiederverwendet werden können.

b) Das nach Beispiel 2a) erhaltene n-Octyl-N-(2-methyl-1-propenyl)-carbamat wird bei 200 °C/2,7 mbar in einem aus einem elektrisch beheizten Quarzrohr bestehenden Verdampfer kontinuierlich verdampft und die Dämpfe bei 450 °C/2,7 mbar durch ein mit 220 Volumenteilen $V_2A$-Maschendrahtringen (∅ 3 mm) gefülltes, elektrisch beheiztes Quarzrohr geführt. Aus den austretenden Dämpfen werden zunächst bei ca 10 °C (Wasserkühlung) Octanol und unumgesetzter Stoff IV auskondensiert, während sich das gebildete 2-Methyl-1-propenylisocyanat bei − 78 °C in einer nachgeschalteten Trockeneiskühlfalle abscheidet. Nach insgesamt 14 Stunden Reaktionszeit erhält man durch fraktionierende Destillation 90,1 Teile Stoff IV (69,8 % Umsatz) und 83 Teile 2-Methyl-1-propenylisocyanat (65,1 % Ausbeute, bezogen auf ein- bzw. 93,3 % Ausbeute, bezogen auf umgesetztes n-Octyl-N-(2-methyl-1-propenyl)-carbamat) vom Kp 58 °C/200 mbar.

Beispiel 3

a) Zu einer Lösung von 750 Teilen Methylcarbamat in 2 000 Volumenteilen Diethylether werden bei 25 °C nacheinander 430 Teile n-Valeraldehyd und 5 Teile p-Toluolsulfonsäure zugegeben. Nach einer Stunde Reaktion unter Temperaturanstieg auf 40 °C erhält man durch Absaugen und anschließende fraktionierte Destillation des Festgutes in Gegenwart von 15 Teilen Kaliumcarbonat und 2 Teilen Hydrochinon 559,1 Teile Methyl-N-(1-pentenyl)-carbamat (78,2 % Ausbeute, bezogen auf eingesetzten n-Valeraldehyd) als cis-trans-Isomerengemisch vom Kp 97-102 °C/16 mbar sowie 352,5 Teile Methylcarbamat, das wiederverwendet werden kann.

b) Das nach Beispiel 3a) erhaltene Methyl-N-(1-pentenyl)-carbamat wird bei 160-170 °C/80 mbar kontinuierlich in einem aus einem elektrisch beheizten Quarzrohr bestehenden Verdampfer verdampft und die Dämpfe bei 450 °C/80 mbar durch ein mit 140 Volumenteilen $V_2A$-Maschendrahtringen (∅ 3 mm) gefülltes, elektrisch beheiztes Quarzrohr geführt. Aus den austretenden Dämpfen werden zunächst bei ca 10 °C (Wasserkühlung) 1-Pentenylisocyanat und unumgesetzter Stoff IV auskondensiert, während sich das gebildete Methanol bei − 78 °C in einer nachgeschalteten Trokkeneiskühlfalle abscheidet. Nach insgesamt 23 Stunden Reaktionszeit erhält man durch fraktionierte Destillation des Kondensats 221,4 Teile Stoff IV (60,4 % Umsatz) und 188,8 Teile 1-Pentenylisocyanat (43,5 % Ausbeute, bezogen auf ein- bzw. 70 % Ausbeute, bezogen auf umgesetztes Methyl-N-(1-pentenyl)-carbamat) vom Kp 52 bis 55 °C/60 mbar.

**Anspruch**

Verfahren zur Herstellung von 1-Alkenylisocyanaten der Formel

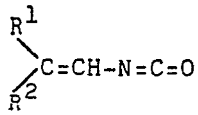

(I)

worin $R^1$ und $R^2$ gleich oder verschieden sein können und jeweils ein Wasserstoffatom, ein Halogenatom, einen aliphatischen, cycloaliphatischen, araliphatischen oder aromatischen Rest bedeuten, $R^1$ und $R^2$ zusammen mit dem benachbarten Kohlenstoffatom auch Glieder eines Ringes bezeichnen, dadurch gekennzeichnet, daβ man einen Aldehyd der Formel

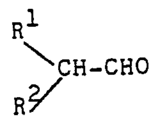

(II)

worin $R^1$ und $R^2$ die vorgenannte Bedeutung besitzen, mit einem Carbamat der Formel

$$H_2N—CO_2R^3 \qquad (III)$$

worin $R^3$ einen aliphatischen, cycloaliphatischen oder araliphatischen Rest bedeutet, zu einem N-(1-Alkenyl)-carbamat der Formel

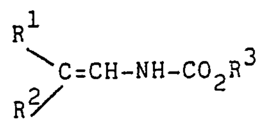

(IV)

worin $R^1$, $R^2$ und $R^3$ die vorgenannten Bedeutungen besitzen, umsetzt und den Stoff IV bei Temperaturen von 250 bis 600 °C erhitzt.

**Claim**

A process for the preparation of a 1-alkenyl isocyanate of the formula

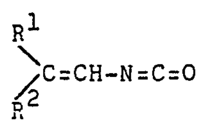

(I)

where $R^1$ and $R^2$ can be identical or different and each is hydrogen, halogen or an aliphatic, cycloaliphatic, araliphatic or aromatic radical, or $R^1$ and $R^2$, together with the adjacent carbon, are members of a ring, wherein an aldehyde of the formula

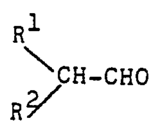

(II)

where $R^1$ and $R^2$ have the above meanings, is reacted with a carbamate of the formula

$$H_2N—CO_2R_3 \qquad (III)$$

where $R^3$ is an aliphatic, cycloaliphatic or araliphatic radical, to give an N-(1-alkenyl)-carbamate of the formula

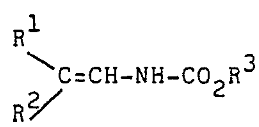

(IV)

where $R^1$, $R^2$ and $R^3$ have the above meanings, and the compound IV is heated at from 250 to 600 °C.

**Revendication**

Procédé de préparation d'isocyanates de 1-alcényle de la formule

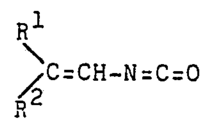

(I)

dans laquelle $R^1$ et $R^2$, qui peuvent être identiques ou différents, désignent chacun un atome d'hydrogène ou d'halogène ou un groupe aliphatique, cyclo-aliphatique, araliphatique ou aromatique, $R^1$ et $R^2$ pouvant aussi constituer avec l'atome de carbone adjacent des éléments d'un cycle, caractérisé en ce que l'on transforme un aldéhyde de la formule

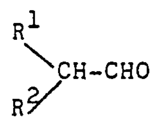

(II)

dans laquelle $R^1$ et $R^2$ possèdent la signification définie, par réaction avec un carbamate de la formule

$$H_2N—CO_2R^3 \qquad (III)$$

dans laquelle $R^3$ désigne un groupe aliphatique, cyclo-aliphatique ou araliphatique, en un carbamate de N-(1-alcényle) de la formule

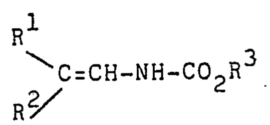

(IV)

dans laquelle $R^1$, $R^2$ et $R^3$ possèdent les significations définies, puis on chauffe le composé IV à des températures de 250 à 600 °C.